# EUROPÄISCHE PATENTSCHRIFT

(11) **EP 0 650 054 B1**
(45) Veröffentlichungstag und Bekanntmachung des Hinweises auf die Patenterteilung: **30.08.2000**
(21) Anmeldenummer: 94116322.2
(22) Anmeldetag: 17.10.1994
(51) Int. Cl.: G01N 33/543, G01N 33/558, G01N 33/94

(54) **Verfahren und Vorrichtung zur Gewinnung und zum Nachweis von immunologisch aktiven Substanzen aus der Gasphase**
Process and device for obtaining and detecting immunologically reactive substances from the gas phase
Procédé et dispositif d'obtention et de détection de substances réactives immunologiquement d'une phase gazeuse

(30) Priorität: 20.10.1993 DE 4335780; 22.07.1994 DE 4425963
(43) Veröffentlichungstag der Anmeldung: 26.04.1995
(73) Patentinhaber: SECURETEC GmbH, 85521 Ottobrunn (DE)
(72) Erfinder: Klein, Christian, Dr., D-82362 Weilheim (DE); Josel, Hans-Peter, Dr., D-82362 Weilheim (DE); Herrmann, Rupert, Dr., D-82362 Weilheim (DE); Maier, Josef, D-82362 Weilheim (DE); Ertl, Harald, D-82538 Gelting (DE); Oberpriller, Helmut, D-83620 Westerhamm (DE); Hilpert, Reinhold, Dr., D-82272 Moorenweis (DE); Binder, Florian, Dr., D-83278 Traunstein (DE); Ritter, Josef, Dr., D-80339 München (DE)

(56) Entgegenhaltungen:
- EP-A- 0 139 373
- EP-A- 0 374 684
- EP-A- 0 520 202
- WO-A-87/07724
- JOURNAL OF THE AMERICAN CHEMICAL SOCIETY, Bd. 108, Oktober 1986 Seiten 5444-5447, NGEH-NGWAINBI ET AL. 'Parathion antibodies on piezoelectric crystals'

## Beschreibung

Gegenstand der Erfindung ist ein Verfahren und eine Vorrichtung zur Gewinnung und zum Nachweis von immunologisch aktiven Substanzen (wie z.B. Drogen) aus der Gasphase über ein immunologisches Bestimmungsverfahren.

Der Nachweis von immunologisch aktiven Substanzen aus der Gasphase ist insbesondere zum Nachweis von Drogen wie z.B. Cocain und Cannabinoide von großer Bedeutung.

Bisher werden mit Hilfe von verschiedenen Techniken (Röntgenanlagen, GC-MS-Kopplung, GC mit Chemilumineszenzdetektor) ortsgebundene Untersuchungen auf das Vorhandensein illegaler Drogen, z.B. in Gepäckstücken, durchgeführt. Einem verbreiteten Einsatz dieser Techniken stehen jedoch Größe und Gewicht dieser Anlagen und die hohen Kosten entgegen. Zum mobilen Einsatz eignen sich prinzipiell Handgeräte, die auf der Ionenbeweglichkeitsspektroskopie (IMS) basieren. Hier treten jedoch Probleme sowohl hinsichtlich Selektivität als auch Empfindlichkeit auf. Außerdem sind derartige Geräte nur zum Nachweis partikulär vorhandener Kontaminationen geeignet. Als einziges derzeit in der Praxis taugliches Verfahren zum selektiven Nachweis von Betäubungsmitteln in der Gasphase wird der Einsatz von Spürhunden angesehen. Der Nachteil des Einsatzes von Spürhunden liegt jedoch in geringer Einsatzdauer, dem Problem der Ablenkung, der Überdeckung durch Reizstoffe und in den hohen Kosten. Ein mobil einsetzbares, zur selektiven Detektion von Betäubungsmitteln unter unterschiedlichen Einsatzbedingungen geeignetes Gerät würde demnach einen enormen Fortschritt für die Bekämpfung des illegalen Drogenhandels darstellen.

Biosensoren, die das Prinzip einer immunologischen Reaktion zwischen dem Analyten und im Biosensor enthaltenen Bindepartnern ausnützen, wären aufgrund der hohen Selektivität und Spezifität der immunologischen Reaktionen für derartige Aufgaben potentiell geeignet. Ein derartiger Biosensor ist von Ngen-Ngwainbi, J., et al. in J. Am. Chem. Soc. 108(1986) 5444-5447 beschrieben. Dort wird als reaktive Komponente des Sensors ein Antikörper gegen ein Stoffwechselprodukt des Cocains (Benzoylecgonin) als Transducer Piezodickenscherungsschwinger mit einer Resonanzfrequenz von 9MHz eingesetzt. Die Antikörper werden durch rein physikalische Adsorption auf der Oberfläche des Sensors immobilisiert. Es wird eine untere Empfindlichkeit von 0,5ppb entsprechend 2 x 10⁻¹¹ mol/l Gasphase (für Cocain und Cocain-HCl) angegeben. Da die Massensensivität des verwendeten Schwingers sehr gering ist, kann mit einem derartigen Gerät keine praxisrelevante Leistungsfähigkeit erreicht werden.

Ein weiteres Verfahren ist in der JP-A-0374460 beschrieben. Dort werden Drogenmoleküle aus der Gasphase an eine Polyethersulfonmembran adsorbiert, eluiert und im Eluat durch eine immunologische Reaktion nachgewiesen. Auch hier ist die Empfindlichkeit sehr gering.

Ein weiteres Verfahren wird in der DE-A 41 21 493 beschrieben. Danach liegen in einem permeablen, undurchsichtigen Teilbereich eines Trägers immobilisierter Antikörper und markierter Tracer (entspricht dem Analyten) aneinander gebunden vor. Der Analyt wird zugegeben und verdrängt markierten Tracer von seiner Bindung an den Antikörper. Der freie markierte Tracer diffundiert dann in einen transparenten Teilbereich des Trägers. Das Fortschreiten der Tracerdiffusion kann an einer Verfärbung erkannt werden, da als Markierung im Tracer ein Farbstoffverwendet wird. Die Empfindlichkeit dieses Verfahrens ist jedoch ebenfalls nur sehr gering.

Die für einen erfolgreichen Nachweis von Drogen aus dem Gasraum erforderlichen unteren Nachweisgrenzen werden auf diese Weise nicht erreicht.

So hat z.B. Cocain eine Sättigungskonzentration von 6x 10⁻¹²mol/l Gasphase bei 20 °C. In der Praxis treten jedoch wesentlich niedrigere Konzentrationen auf so daß die untere Nachweisgrenze eines Verfahrens zum Nachweis bei 10⁻¹³ bis 10⁻¹⁵ mol/l Gasphase liegt.

Da die Messung solch niedriger Konzentrationen direkt aus der Gasphase nicht möglich ist, ist eine Vorrichtung zur Bestimmung immunologischer aktiver Substanzen aus der Gasphase zweckmäßigerweise aus einer Adsorptions- und einer Detektionseinheit aufgebaut.

Bei der Bekämpfung der Drogenkriminalität ist es z.B. notwendig, neben in der Gasphase frei vorkommenden Drogenmolekülen auch partikelförmige oder an Partikel gebundene Drogen nachzuweisen (BKA ed. Internationales Symposium Detektion von Rauschgift, Wiesbaden 1991).

Aufgabe der Erfindung ist die Bereitstellung eines Verfahrens zur Gewinnung von immunologisch aktiven Substanzen aus der Gasphase, durch das die nachzuweisende Substanz aus der Gasphase in eine gegebenenfalls für nachfolgende Nachweisreaktionen geeignete Form überführt wird.

Eine weitere Aufgabe der Erfindung ist die Bereitstellung eines Verfahrens zur Bestimmung von immunologisch aktiven Substanzen aus der Gasphase, das eine hohe Empfindlichkeit zeigt und eine schnelle Adsorption bzw. Adsorption und Bestimmung erlaubt.

Immunologisch aktive Substanzen aus der Gasphase sind sowohl freie in der Gasphase vorkommende Substanzen, Partikel dieser Substanzen oder Substanzen an Partikel gebunden, die aus der Gasphase gewinnbar sind.

Gegenstand der Erfindung ist ein Verfahren zur Gewinnung eines in einer Gasphase enthaltenen Analyten durch immunologische Bindung des Analyten an einen in einer gas-und flüssigkeitspermeablen ersten Trägermatrix enthaltenen Bindepartner des Analyten, welches dadurch gekennzeichnet ist, daß
a) die analythaltige Gasphase mit der ersten Trägermatrix (Immunadsorber) in Kontakt gebracht wird,
b) der Analyt an den in der ersten Matrix enthaltenen und nicht an die Matrix gebundenen ersten Bindepartner gebunden wird und
c) der Komplex aus Analyt und erstem Bindepartner aus der ersten Matrix eluiert wird und
d) gegebenenfalls der Analyt aus dem Komplex freigesetzt wird.

Ein weiterer Gegenstand der Erfindung ist ein Verfahren zum immunologischen Nachweis eines in einer Gasphase enthaltenen Analyten durch immunologische Bindung des Analyten an einen in einer gas- und flüssigkeitspermeablen ersten Trägermatrix enthaltenen Bindepartner des Analyten, welches dadurch gekennzeichnet ist, daß
a) die analythaltige Gasphase mit der ersten Trägermatrix (Immunadsorber) in Kontakt gebracht wird,
b) der Analyt an den in der ersten Matrix enthaltenen und nicht an die Matrix gebundenen ersten Bindepartner gebunden wird,
c) das Gemisch aus dem Komplex aus Analyt und erstem Bindepartner zusammen mit gegebenenfalls überschüssigem freiem ersten Bindepartner aus der ersten Matrix eluiert werden und
d) der eluierte Komplex oder der freie erste Bindepartner als Maß für die Anwesenheit oder Menge des Analyten bestimmt wird.

Als Bindepartner des Analyten werden zweckmäßig Antikörper, die monoklonal oder polyklonal sein können, bzw. deren Fragmente (z.B. Fab, Fab') verwendet. Ebenso geeignet sind die im Körper vorhandenen Rezeptoren, an die diese Drogen binden, z.B. ein Cannabinoid-Rezeptor (Matsuda et al, Nature 346 (1990) 561-564).

Bevorzugt ist der erste Bindepartner markiert, z.B. enzymmarkiert oder direktmarkiert und kann über diese Markierung bestimmt werden. Die Trägermatrices bestehen bevorzugt aus hydrophilem oder hygroskopischem Material, z.B. auf Basis von Zellulose, modifizierter Zellulose wie Zellulosenitrat oder Zelluloseacetat, hydroxyalkylierter Zellulose oder mit Mitteln wie z.B. Epichlorhydrin vernetzter, modifizierter und unmodifizierter Zellulose. Ebenso geeignet sind Glasfasermatrices und Matrices aus Polyester. Diese Materialien können entweder weder allein oder in Kombination mit anderen Verschnittmaterialien verwendet werden, wobei vorzugsweise der hydrophile Anteil in der Trägermatrix überwiegt.

In einer alternativen Ausführungsform ist der erste Bindepartner unmarkiert. In diesem Fall wird die eluierte Menge an erstem Bindepartner an einen markierten zweiten Bindepartner gebunden und die so gebundene Markierung als Maß für die Menge des vorhandenen Analyten bestimmt. Bevorzugt erfolgt die Bindung des ersten Bindepartners an den zweiten Bindepartner in einer zweiten Trägermatrix und die Bestimmung der Markierung in einer dritten Trägermatrix.

Vorzugsweise wird das erste Trägermaterial und ggf. die weiteren Trägermaterialien partikulär (z.B. perlförmig, vgl. DD-A 296 005) oder aus Fasern aufgebaut, wie z.B. aus Filterpapieren auf Zellulosebasis (EP-A 374 684, EP-A 0 470 565). Weitere Materialien, die für den Aufbau des Testträgers verwendet werden können, sind in der EP-A 0 374 684, EP-A 0 353 570 und EP-A 0 353 501 beschrieben.

Die erste Trägermatrix muß gasdurchlässig sein, um eine Anreicherung der immunologisch aktiven Substanz aus der Gasphase zu ermöglichen. Die Gasdurchlässigkeiten bei technisch einfach zu realisierenden Druckabfällen über dem Adsorber (200 - 500 mbar) liegen zweckmäßig zwischen 1 ml/min und 100 l/min, besser jedoch zwischen 10 ml/min und 10 l/min, vorzugsweise zwischen 100 ml/min und 20 l/min und besonders bevorzugt zwischen 500 ml/min und 10 l/min.

Die Anforderung an die erste Matrix (Immunadsorber) besteht neben der Rückhaltung des Analyten im wesentlichen darin, daß die Immunreaktion zwischen dem Bindepartner und der immunologisch aktiven Substanz gegeben ist, z.B. durch Diffusion. Dazu kann die erste Matrix entweder bereits vor Beginn der Adsorption die hierfür erforderlichen Flüssigkeiten enthalten oder es kann erst durch Zugabe von Flüssigkeit eine Immunreaktion ermöglicht werden. Im letztgenannten Fall wird der Analyt in einem ersten Schrift an eine im wesentlichen trockene Matrix unspezifisch adsorbiert. In einem zweiten Schritt wird Flüssigkeit zugegeben, wodurch die Immunreaktion zwischen Analyt und markiertem Bindepartner erfolgt.

Als Flüssigkeit in der ersten Matrix wird bevorzugt Wasser verwendet. Enthält die erste Matrix bereits vor der Adsorption Flüssigkeit, so liegt der Gehalt zwischen 10 und 90% des Gesamtgewichtes der ersten Matrix. Das verwendete Wasser kann jedoch einen Anteil von bis zu 30Gew.-% organischer Substanzen als Lösevermittler enthalten (wie z.B. Dimethylsulfoxid, Glycerin).

Es ist bevorzugt 0,01 bis 1% Detergenzien (z.B. Tween 20, Tween 80, Octylglucosid, Polydocanol und/oder Synperonic, z.B. F 68) zur Löslichkeitsverbesserung der Bindepartner zuzusetzen.

Erfolgt nach der Adsorption ein Nachweis des Analyten, der auf der Detektion eines markierten Bindepartners beruht, so können als Markierung alle für Immunoassays beschriebenen Markierungen erfindungsgemäß verwendet werden. Dies sind beispielsweise Enzymmarkierungen, Fluoreszenzfarbstoffmarkierungen, radioaktive Markierungen, Markierungen mit Gold, lumineszenzfähigen Molekülen oder Selendioxid oder Markierungen mit gefärbten Polymeren, wie z.B. Latexpartikel.

Es ist jedoch erfindungsgemäß bevorzugt, als Markierung ein Enzym zu verwenden und dieses über eine enzymatische Farbbildungsreaktion nachzuweisen Besonders bevorzugte Enzyme sind hierbei β-D-Galactosidase, alkalische Phosphatase oder Peroxidase. Die beispielsweise über die Substrate Chlorphenolrot-β-D-Galactosid, p-Nitrophenol-phosphat AMPPD (di-Natrium 3-(Methoxyspiro{1,2-dioxetan-3,2 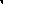 -tricyclo[3.3.1.1.3,7]decan}phenylphosphat) oder ABTS ® entsprechend nachgewiesen werden. In einer weiteren bevorzugten Ausführungsform der Erfindung verwendet man als Markierung einen Fluoreszenzfarbstoff, dessen Fluoreszenzquantenausbeute oder Depolarisationsvermögen sich durch die Bindung an den Antikörper in Abhängigkeit von der Konzentration des in der Probenflüssigkeit enthaltenen Analyten ändert. Besonders bevorzugt ist es, bei Verwendung von Enzymmarkierungen zur Erhöhung der Sensitivität bei der Detektion der Markierung ein Verstärkungssystem über eine Amplifikationsreaktion wie beispielsweise in DD222896, DD222897 und DD280790 beschrieben zu verwenden.

Besonders bevorzugt sind Markierungen, die die Detektion einer Lumineszenz ermöglichen, z.B. bei Enzymmarkierung alkalischer Phosphate in Kombination mit z.B. AMIPPD (di-Natrium 3-(Methoxyspiro{1,2-dioxetan-3,2 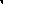 -tricyclo[3.3.1.1.3,7]decan}phenylphosphat) oder CSBD (di-Natrium 3-(Methoxyspiro{1,2-dioxetan-3-2 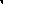 -(5-chlor)-tricyclo[3.3.1.1.3,7]decan}phenylphosphat oder Peroxidase mit Luminol, bei lumineszenzfähigen Molekülen Aequorin (Biochemistry 1992, Vol. 31, Seite 1433 - 1442), dessen Lumineszenz durch Calcium-Ionen ausgelöst wird oder Acridiniumester, deren Lumineszenz durch Peroxide ausgelöst werden.

Die erfindungsgemäß verwendeten Antikörper sind nach den bekannten Methoden der Immunisierung von geeigneten Tieren mit einem entsprechenden Immunogen herstellbar. Die Antikörper werden nach Immunisierung gewonnen und diejenigen ausgewählt, die eine möglichst hohe Spezifität und Affinität zum Analyten besitzen. Auch die Herstellung von monoklonalen Antikörpern und Antikörperfragmenten ist dem Fachmann bekannt.

Die Durchführung der Bestimmung erfolgt in der Weise, daß der Komplex aus Analyt und erstem Bindepartner sowie freier erster Bindepartner aus der ersten Matrix im ersten Schritt eluiert werden. Der Analyt kann in diesem Eluat mit allen bekannten Immunoassayverfahren bestimmt werden.

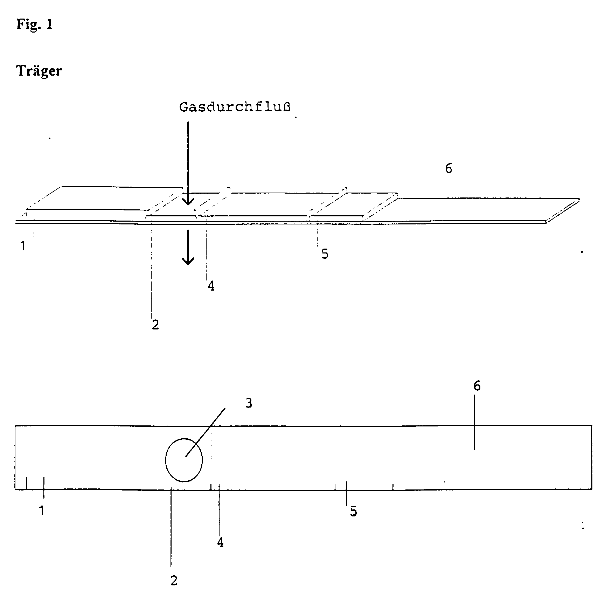

In einer bevorzugten Ausführungsform besteht der Träger im wesentlichen aus vier Zonen (Fig. 1):
1. erste Trägermatrix (Immunadsorber), enthält nicht an den Träger gebundenen ersten Bindungspartner (Konjugat aus Antikörper und Markierung);
2. eine zweite Trägermatrix (Abfangmatrix), in der nicht analytgebundenes Konjugat an immobilisiertes Analytanalogon gebunden wird und gegebenenfalls bestimmt werden kann.
3. dritte Trägermatrix (Nachweisfeld), in der bevorzugt die Bestimmung der Markierung erfolgt.
4. Vor der ersten Trägermatrix kann gegebenenfalls ein Vorratsbehälter mit der für die Elution geeigneten Flüssigkeit angeordnet sein. Vorzugsweise handelt es sich hierbei um ein Trägervlies.

Die Durchführung der Bestimmung erfolgt in der Weise, daß durch Herbeiführung einer Druckdifferenz das zu prüfende Gas in die erste Trägermatrix gesaugt oder gepumpt wird. Nach ausreichender Anreicherung des Analyten auf dem Immunadsorber und ggf. Bildung des Komplexes zwischen Analyt und Bindepartner wird die Elutionsflüssigkeit aus dem Vorratsbehälter beispielsweise durch Druckanwendung in die erste Trägermatrix überführt. Auch bei diesem Schritt kann die Komplexbildung zwischen Analyt und Bindepartner erfolgen. Die löslichen Komponenten der ersten Trägermatrix eluieren in die zweite Trägermatrix (Abfangmatrix). Dort wird die Menge des Rezeptors, welche nicht mit dem Analyten eine immunologische Bindung eingegangen ist, an überschüssige, trägergebundene Analytanaloga gebunden. Das Konjugat aus Analyt und spezifischem Rezeptor wandert weiter in die Dritträgermatrix, in der dann die Markierung als Maß für Anwesenheit oder Menge des Analyten nachgewiesen wird. Die Markierung kann auch in der Abfangmatrix als Maß für die Menge des Analyten bestimmt werden.

Wird beispielsweise auf der ersten Trägermatrix (Immunadsorber) ein Konjugat aus einem Antikörper und Acridiniumester eingesetzt, so erfolgt die Detektion im Nachweisfeld durch Messung einer durch alkalisches Peroxid ausgelösten Lumineszenz. Wird in der ersten Trägermatrix ein Konjugat aus einem Antikörper und alkalischer Phosphatase verwendet, so erfolgt nach Diffusion in die zweite Trägermatrix (Abfangmatrix) die Messung einer Lumineszenz mit einem luminogenen AP-Substrat wie Lumiphos (di-Natrium 3-(Methoxyspiro{1,2-.dioxetan-3,2 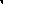 -tricyclo [3.3.1.1.3,7]decan}phenylphosphat) oder einem chromogenen AP-Substrat (X-Gal, NBT) im Nachweisfeld. Wird als Konjugat ein Konjugat aus Antikörper und Goldsol verwendet, so erfolgt die Detektion in der dritten Trägermatrix visuell nach Chromatographie über eine Abfangmatrix. Derartige Abfangmatrices sind z.B. beschrieben in der EP-A 0052769, EP-A 0167171 und EP-A 0470565.

Wird als Konjugat ein Konjugat aus einem Antikörper und Aequorin verwendet, erfolgt die Messung einer Lumineszenz in der dritten Trägermatrix durch Calcium-Ionen, mit denen die dritte Trägermatrix getränkt wurde oder durch Zugabe von Calcium-Ionen.

In einer weiteren Ausführungsform wird das aus der ersten Matrix eluierte Gemisch aus Antikörper-Analytkomplex und überschüssigem unmarkierten Antikörper mit einer Festphase, die mit Analyt beschichtet ist, inkubiert und gewaschen. An der Festphase werden Antikörper abgefangen. Danach wird [Bit markierten Antikörpern, die gegen den Analytantikörper (vorzugsweise gegen dessen konstanten Teil) gerichtet sind, inkubiert und gewaschen und die Menge an gebundenem und von der ersten Matrix eluierten freien Antikörper direkt gemessen durch eine Substratreaktion, z.B. mit ABTS® bei Enzymmarkierung, nachgewiesen. Wird anstelle des Analytantikörpers ein Konjugat aus Analytantikörper und Enzym (z.B. Peroxidase) verwendet, kann der Inkubations- und Waschschritt mit dem sekundären Nachweiskonjugat aus Peroxidase und Antikörper gegen den Analytantikörper eingespart werden. Ebenso ist es möglich, anstelle des AnalytantikörperEnzymkonjugats ein Analytantikörper-Acridiniumester-Konjugat zu verwenden und durch Reaktion mit alkalischer Peroxidlösung luminometrisch nachzuweisen.

Die Konjugate aus Antikörper und Markierung können auf die Trägermaterialien durch einfaches Benetzen aufgebracht werden. Ebenso möglich ist es, eine Lösung des Konjugats auf den getrockneten Träger aufzugeben, die Lösung aufsaugen zu lassen und danach überschüssige Flüssigkeit durch Abtropfen, Abzentrifugieren oder kurzes Durchsaugen von Luft abzutrennen. Das Konjugat wird vorzugsweise in einer gepufferten Lösung wie PBS oder andere gebräuchliche Puffer pH 5 - 9 auf den Träger aufgebracht. Diese Lösung kann zusätzlich zur Verbesserung der Durchdringung des Trägers und zur Stabilisierung nicht-denaturierende Detergenzien, wie Tween 20, Tween 80, Octylglucosid, Polydocanol, Synperonic F 68, oder organische Lösungsmittel, wie Glycerin DMSO, und/oder Polyethylenglycole und/oder Proteinzusätze, wie Rinderserumalbum oder unspezifisches IgG, enthalten.

Zur Elution werden zweckmäßig gepufferte Lösungen wie PBS oder andere gebräuchliche Puffer im pH-Bereich zwischen 5 und 9 verwendet.

Ein weiterer Gegenstand der Erfindung ist eine Vorrichtung zur Gewinnung und/oder zum Nachweis einer in einer Gasphase enthaltenen immunologisch aktiven Substanz, enthaltend
a) eine gas- und flüssigkeitspermeable Trägermatrix, in der ein erster Bindepartner des Analyten in eluierbarer Form enthalten ist,
b) ein Auffangsystem für ein solches Eluat, in welchem der Komplex aus Analyt und erstem Bindepartner oder ungebundener erster Bindepartner isoliert und gegebenenfalls in einer Folgereaktion bestimmt werden kann.

Dabei kann die Matrix des erfindungsgemäßen Immunadsorbers flächig (als Vlies oder Membran) oder in Form tiefer Schichten (z.B. in Säulenform) angeordnet sein. Bei den zur Adsorption in Frage kommenden Volumenströmen 1 ml bis 100l/min, bevorzugt 100ml bis 20 l/min, besonders bevorzugt 500 ml/min bis 10l/min, können im Fall flächiger Matrices runde oder anders geformte Flächen zwischen 2mm² und 100cm² Flächenmaß und einer Tiefe zwischen 0,1 mm und 10mm eingesetzt werden. Bevorzugt verwendet werden Matrices mit einer Fläche zwischen 0,2 und 5cm² Flächenmaß und 0,5 bis 2mm Tiefe. Im Fall tieferer Schichten werden bevorzugt säulenartige Geometrien verwendet, wobei der Durchmesser zwischen 3mm² und 25cm² und Schichtdicken zwischen 3mm und 20cm eingesetzt werden können. Bevorzugt wird ein Bereich zwischen 10mm² und 100mm² im Durchmesser und 1 bis 10cm Schichtdicke. Die Form des Matrixmaterials kann dann kugelförmig, faserförmig, unregelmäßig gekörnt oder auch gelförmig sein.

Das Auffangsystem kann ebenfalls flächig (Trägermatrix), in Säulenform oder als Behälter für kleine Flüssigkeitsmengen ausgebildet sein.

Die Träger bestehen bevorzugt aus mehreren, im wesentlichen nebeneinander angeordneten kapillaraktiven Testfeldern (Trägermatrices), die in Fluidkontakt zueinander stehen, so daß sie eine Flüssigkeitstransportstrecke bilden, entlang der eine Flüsigkeit durch Kapillarkräfte getrieben durch den Immunadsorber zum Nachweisfeld strömt. Die Anzahl der Felder ist an sich unkritisch und hängt davon ab, ob die für die immunologische Reaktion notwendigen Reagenzien auf getrennte oder gemeinsame Felder aufgebracht werden sollen.

Die Testfelder werden in EP-A-0 374 684 beschrieben mit Schmelzkleber auf einer Trägerfolie aufgeklebt. Die Trägerfolie ist dabei für den Gasdurchtritt so gelocht, daß das Testfeld (erste Trägermatrix)jeweils über den Öffnungen zu liegen kommt. Die beklebte Trägerfolie wird anschließend so geschnitten, daß einzelne Träger erhalten werden, bei denen sich die Gasdurchtrittsöffnung jeweils im wesentlichen mittig unter der ersten Trägermatrix befindet.

Figur 1a) und 1b) zeigen ein Beispiel eines Testträgers. Es bedeuten:
1) Vlies zur Aufnahme von Elutionsflüssigkeit
2) Immunadsorbermatrix über einer Durchsaugöffnung (3) der Trägerfolie (6)
3) Durchsaugöffnung
4) Abfangmatrix
5) Nachweismatrix

In einer weiteren Ausführungsform enthält die Vorrichtung zusätzlich mindestens ein Pufferfeld, welche in Kontakt mit Immunadsorber, Abfangmatrix und/oder Nachweisfeld steht. Es enthält Hilfssubstanzen, mit denen die Bedingungen für eine optimale Reaktionsführung (z.B. Ionenstärke, pH-Wert) hergestellt werden. Das Pufferfeld besteht aus porösem Material, vorzugsweise aus einem Vlies auf Cellulose-, Polyester- oder Nylonbasis. Geeignete Trägermaterialien und Verfahren zum Aufbringen des Konjugats sind beispielsweise in EP-A0353570 beschrieben. Besonders geeignet sind poröse Materialien auf Polyester-, Cellulose oder Glasfaserbasis.

Die Immobilisierung des Analytanalogon in der Abfangmatrix kann nach bekannten Verfahren, beispielsweise chemisch oder durch Immunpräzipitation, erfolgen. Vorzugsweise wird jedoch an das Material des Abfangfelds ein biologischer Bindungspartner, wie z.B. (Strept)-Avidin, gebunden und der Antikörper bzw. das erfindungsgemäße Peptid an den anderen biologischen Bindungspartner, beispielsweise Biotin, gebunden zugegeben. Über die Bindung des biologischen Bindungspartners (z.B. Biotin/Streptavidin-Bindung) wird dann die Immobilisierung des Analyt-Analogon bzw. des Antikörpers erreicht. Derartige Verfahren zur Immobilisierung sind beispielsweise in der EP-A 0374684 beschrieben.

Die Nachweiszone kann vorzugsweise gegebenenenfalls teilweise der Abfangmatrix auf-oder unterlegt sein (vgl. EP-A 0353500). Die Nachweiszone enthält vorzugsweise ein Reagenzsystem mit einem für die Markierung geeigneten Nachweissubstrat, das bei Zusammentreffen mit der markierten Komponente einer Veränderung unterliegt, die beobachtet werden kann, oder dient bei Verwendung einer Markierung, die direkt bestimmt werden kann (z.B. Fluoreszenzmarkierung) zum direkten Nachweis des Konjugats. Falls ein Substrat enthalten ist, besteht diese Zone vorzugsweise aus einem auflösbaren Film oder einem Gewebe bzw. Vlies, das hydrophob gesperrt ist. Derartige Träger sind beispielsweise in EP-A 0353501 beschrieben. Dadurch wird erreicht, daß die Probenflüssigkeit nach Erreichen des Feldendes das Substrat aus dem Träger herauslöst und die Nachweisreaktion in Gang gesetzt wird.

Beispiele für nachweisbare Substanzen sind als Drogenmoleküle Cocain, Heroin, Cannabinol, Cannabidiol, Tetrahydrocannabinol, als Sprengstoffe Nitroglycol, Nitroglycerin, Nitropenta, Hexogen, Oktogen, Tetranitromethan, Trinitrotoluol, Trinitrobenzol, Trinitroanisol, Triaminotrinitrotoluol, Hexanitrostilben sowie polycyclische aromatische Kohlenwasserstoffe, polychlorierte Biphenyle, Herbizide und Pestizide (z.B. Atrazin, Parathion, Simazin) und Geruchsstoffe (z.B. Terpineol, Limonen, Caryophyllen, Campher, Farnesol).

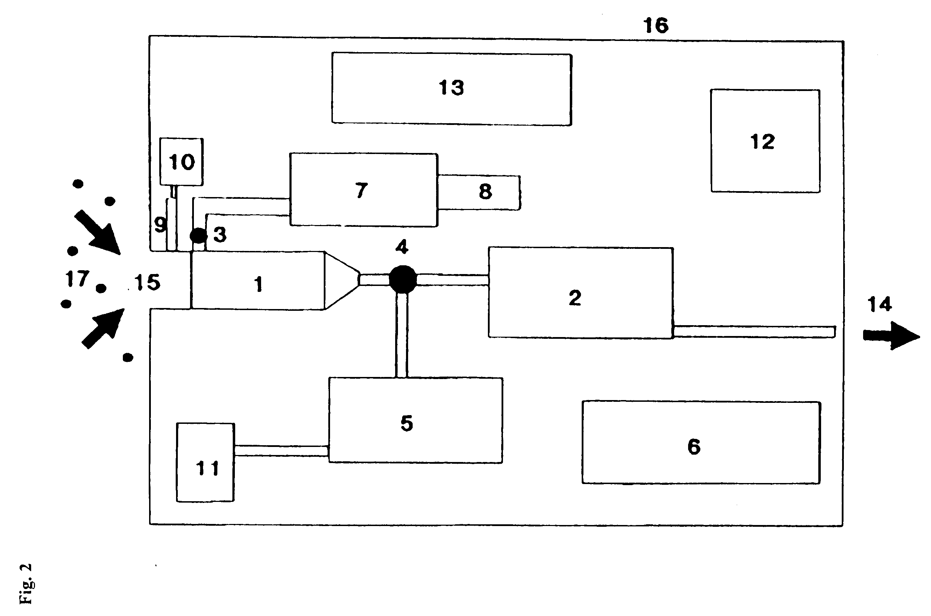

Eine bevorzugte Ausführungsform (Fig. 2) der Vorrichtung besteht aus:
1. erfindungsgemäßer Immunadsorber
2. Vakuumpumpe
3. Ventil
4. Dreiwegeventil
5. Detektionseinheit
6. Signalverarbeitung und -anzeige
7. Vorratsgefäß mit Elutionsmittel
8. Druckpumpe
9. Schieber
10. Antrieb für Schieber
11. Aufnahme für Meßlösungen
12. Energieversorgung
13. Bedienfeld
14. Gasauslaß
15. Eintritt des Probengases
16. Gehäuse
17. gasförmige Probenmoleküle, an Partikel gebundene Probenmoleküle oder partikuläre Probe.

In einem ersten Arbeitstakt wird Probengas, das zu adsorbierende Moleküle 17 enthält, mittels einer Vakuumpumpe 2 durch den Probeneinlaß 15 durch den Immunadsorber 1 gesaugt, wobei das Gas beim Auslaß 14 die Vorrichtung wieder verläßt. Gegebenenfalls werden durch Anblasen oder Bürsten dem Untersuchungsgut anhaftende Probenpartikel oder Partikel, an die Probenmoleküle adsorbiert sind in den Ansauggasstrom gebracht. Hierbei sind Ventil 3 geschlossen, Ventil 4 offen zwischen Immunadsorber und Vakuumpumpe und der Schieber 9 vor dem Probeneinlaß geöffnet. Nach Beaufschlagung des Adsorbers 1 mit der zu untersuchenden Probengasmenge wird mittels des Antriebes 10 der Schieber 9 geschlossen. Anschließend werden gleichzeitig Ventil 3 geöffnet. und Ventil 4 auf Durchlaß zwischen Adsorber und Detektionseinheit geschaltet. Mittels der Druckpumpe 8 wird nun Elutionsmittel aus dem Vorratsbehälter 7 durch den Immunadsorber gefördert. Die Elutionsflüssigkeit, die am Austritt aus dem Adsorber 1 nunmehr die Konjugate zwischen den Analytmolekülen (17) und dem markierten Antikörper und abhängig von der im Probengas enthaltenen Menge an Analyt zusätzlich ungebundenen markierten Antikörper enthält, wird nun einer weiteren Verwendung zugeführt, in diesem Fall einer Detektionseinheit 5, in der mittels nachfolgender (immunologischer) Reaktionen eine Bestimmung der in der Probe vorhanden gewesenen Menge der Analytmoleküle (17) erfolgt. Im Anschluß daran wird wiederum mittels Druckpumpe 8 aus dem Vorratsgefäß 7 Elutionsmittel durch die Anordnung gefördert, wobei eine Spülung des Systems erfolgt und gleichzeitig die verbrauchte Meßlösung einer Aufnahme 11 zugeführt wird. Die Verarbeitung und Anzeige der in der Detektionseinheit erhaltenen Signale erfolgt mittels der Funktionseinheit 6, die Bedienung über das Bedienfeld 13. Alle Komponenten werden über einen austauschbaren Akkumulator 12 mit Energie versorgt und sind in einem Gehäuse 16 angeordnet.

Die Überführung des Antikörper-Antigen-Komplexes aus der ersten Trägermatrix in die weiteren Trägermatrices bzw. Reaktionsgefäße kann vorzugsweise durch mechanisches Ausquetschen des in der ersten Trägermatrix vorhandenen Lösungsmittels, durch Aufbringen und Durchpressen eines zusätzlichen Elutionsmittels unter Druck oder Durchsaugen erfolgen. Ebenso kann das zusätzliche Elutionsmittel über Kapillarkräfte/Konzentrationsgradienten/hydrostatischer Druck auf mechanisch drucklose Weise weitertransportiert werden.

Es ist bevorzugt, das vorhandene Lösungsmittel auszuquetschen oder durch Anlegen eines Drucks oder Vakuums das zusätzliche Elutionsmittel durchzupressen oder durchzuziehen.

### Beispiel 1

### a. Herstellung von Benzoylecgoninmaleimidoethylamid

2.4 g Benzoylecgoninhydrochlorid werden in 200 ml trockenem Acetonitril mit 1 g N-Hydroxysuccinimid und 1.8 g Dicyclohexylcarbodiimid versetzt und 3 Std. gerührt. Vom Niederschlag wird abfiltitriert, das Filtrat eingedampft, in Nitromethan aufgenommen und nochmals filtriert. Nach Abdampfen des Lösungsmittels wird mit Ether verrieben. Man erhält 1.13 g Benzoylecgoninsuccinimidylester. Dieses Produkt wird zusammen mit 0.47 g Maleimidoethylaminhydrochlorid (s. WO 90/15798) in 100 ml trockenem Acetonitril aufgenommen. Man gibt 1.1 g Triethylamin zu und rührt 12 Stunden bei Raumtemperatur. Das Reaktionsgemisch wird eingedampft, in 50 ml Essigester aufgenommen und drei Mal mit Natriumhydrogencarbonatlösung ausgeschüttelt. Die Essigesterphase wird eingedampft und das Produkt durch Aufnahme in 10 ml mit HCl gesättigtem Dioxan in das Hydrochlorid überführt. Man filtriert, wäscht mit Ether und erhält so 1 g Benzoylecgoninmaleimidoethylamidhydrochlorid.

### b. Herstellung des Cocain-Immunogens

300 mg Rinderserumalbumin werden in 25 ml 0.1 mol Kaliumphosphatpuffer pH 8.5 mit 106.6 mg S-Acetylthiopropionsäuresuccinimidylester gelöst in 5 ml Dioxan über 3 Stunden bei Raumtemperatur umgesetzt. Das modifizierte Rinderserumalbumin wird durch Gelchromatograhie über ACA 202 mit 0.1 mol Kaliumphosphatpuffer pH 8.5 von niedermolekularen Reaktionsprodukten abgetrennt. Man erhält eine Lösung von 310.5 mg Produkt in 57.5 ml Kaliumphosphatpuffer pH 8.5.
Eine Lösungsmenge, die 100 mg des modifizierten Rinderserumalbumin entspricht, wird mit 4.7 ml 1 molaren Hydroxylaminlösung umgesetzt. Danach gibt man 49.4 mg Benzoylecgoninmaleimidoethylamidhydrochlorid zu und läßt 12 Stunden bei 4°C reagieren. Das erhaltene Cocain-Immunogen wird durch Gelchromatograhie über ACA 202 mit 0.1 mol Kaliumphosphatpuffer pH 8.5 von niedermolekularen Reaktionsprodukten abgetrennt. Man erhält 95 mg Cocain-Immunogen gelöst in 0.1 molarem Kaliumphosphatpuffer pH 8.5.

### c. Gewinnung von Antikörpern gegen Cocain

10 Schafe wurden mit dem Cocain-Immunogen in komplettem Freundschen Adjuvans immunisiert. Die Dosis betrug jeweils 200 µg Immunogen pro Tier für die erste und jede Folgeimmunisierung. Die Immunisierungen erfolgten in monatlichen Abständen. Die erhaltenen Seren wurden in einem Mikrotiterplatten-Assay auf die Anwesenheit von Antikörpern gegen Cocain untersucht. Dazu wurden mit Streptavidin beschichtete Mikrotiterplatten mit Benzoylecgonin-[N'-biotinylaminocaproyl-(3,6-dioxa-8-aminooctyl)amid], hergestellt aus Benzoylecgoninsuccinimidylester und N-(Biotinylaminocaproyl)-1,8-diamino-3,6-dioxaoctan, inkubiert und gewaschen, danach mit den zu untersuchenden Seren inkubiert, wiederum gewaschen und zur Detektion mit einem Konjugat aus Peroxidase und einem Kaninchen-Anti-Schaf-Immunglobulin inkubiert, gewaschen und mit Substrat versetzt. Analog Beispiel 12 aus EP A 0 547 029 wurden die relativen Affinitäten zu Cocain bestimmt. Seren aus S 4987 mit einer guten Affinität gegenüber Cocain wurden für weitere Untersuchungen ausgewählt.

### d. Herstellung von Konjugaten aus Antikörpern gegen Cocain und alkalischer Phosphatase

Polyklonaler Antikörper gegen Cocain vom Schaf, DE-gereinigt (PAK〈Cocain〉S-IGG(DE)) wird aus delipidiertem Rohserum (Schaf) nach dem Fachmann bekannten Verfahren mittels Ammonsulfatfällung und DEAE-Sepharose-Chromatographie isoliert.

### Immunsorptive Reinigung von PAK〈BZE〉S-IGG(DE)

### Herstellung eines Cocain-Immunadsorbers

Das Cocain-Polyhapten aus Beispiel 1 e.(ohne Biotinylierungsschritt) wird an ein Glutardialdehyd-aktiviertes Affinitätsadsorbens (aktiviertes Spherosil; Boehringer Mannheim, Bestell-Nr. 665 525) entsprechend den Angaben des Herstellers gebunden.

### Immunsorption

(PAK〈Cocain〉S-IGG(DE)) wird gegen PBS/Azid (50 mmol/l Kaliumphosphat pH 7.5, 150 mmol/l NaCl, 0.1 % Na-Azid) dialysiert und anschließend innerhalb 2 Std. bei Raumtemperatur über eine geeignet dimensionierte Adsorbens-Säule (abhängig von Bindekapazität des Adsorbens und Titer des IGG(DE)) gegeben. Nach Auswaschen des nicht gebundenen Proteins mit PBS/Azid wird der gebundene Antikörper mit 1M Propionsäure bei Raumtemperatur eluiert. Das Eluat wird gegen 30 mM Natriumphosphat-Puffer pH 7.1 dialysiert.

### Herstellung von PAK〈BZE〉S-IGG(IS)-AP Konjugaten

### Aktivierung des IGG

Das immunsorptiv gereinigte IGG wird bei einer Konzentration von 10 mg Protein / ml in 30 mM Natriumphosphat-Puffer pH 7.1 mit 5 fach molarem Überschuß an Maleimido-hexanoyl-N-hydroxy-succinimidester (MHS) bei 25° C 1 Stunde inkubiert. Der Ansatz wird durch Zugabe von 100 fach molarem Überschuß an L-Lysin/HCl gegenüber MHS gestoppt und gegen 10 mM Kaliumphosphat, 50 mM NaCl, 10 mM MgCl₂, pH 6.1 dialysiert.

### Aktivierung von alkalischer Phosphatase

Die alkalische Phosphatase (EIA-Qualität, Boehringer Mannheim, Bestell-Nr. 567 744) wird bei einer Konzentration von 10 mg Protein / ml in 30 mM Triäthanolamin, 3M NaCl, 0.1 mM ZnCl₂, 1 mM MgCl₂, pH 7.0 mit 30 fach molarem Uberschuß an Succinimidyl-acetylthiopropionat (SATP) bei 25°C 1 Stunde inkubiert. Der Ansatz wird durch Zugabe von L-Lysin/HCl ad 10 mM gestoppt und gegen 10 mM Kaliumphosphat, 50 mM NaCl, pH 7.5 dialysiert. Zur Deacetylierung der geschützten SH-Gruppe wird 1M Hydroxylamin-Lösung pH 7.5 ad 20 mM und 0.1M EDTA-Lösung ad 0.5 mM zugesetzt und 15 min bei 25°C inkubiert. Die aktivierte AP-Lösung wird sofort für die Kopplung eingesetzt.

### Kopplung

Die Lösungen der aktivierten AP sowie des aktivierten IGG werden äquimolar gemischt, der pH auf 6.8-7.0 und die AP-Konzentration mit bidest. Wasser auf 5 mg/ml eingestellt. Nach 3 Std. Reaktion bei 25°C wird der Ansatz durch sequentielle Zugabe von N-Ethylmaleimid (ad 5 mM, 30 min, 25°C) und 1M Hydroxylamin-Lösung pH 7.5 (ad 20 mM. 1 Std. 25°C ) abgestoppt. Die Konjugat-Lösung wird gegen 50 mM Triäthanolamin/HCl, 150 mM NaCl, 1 mM MgCl₂, 0.1 mM ZnCl₂, pH 7.6 dialysiert und ad 10 mg/ml Rinderserumalbumin und 3 M NaCl aufgestockt.

### e. Herstellung eines biotinylierten Cocain-Polyhaptens

Kaninchen-IgG wird bei einer Konzentration von 25 mg/ml in Phospatpuffer pH 8 mit der 6-fach molaren Menge S-Acetylthiopropionsäuresuccinimidylester, gelöst in Dimethylsulfoxid, umgesetzt. Nach 1 Stunde bei 25 °C wird die Reaktion durch Zugabe einer Lösung von 1 mol/l Lysin gestoppt. Es folgt Dialyse gegen 0.1 mol/l Kaliumphosphatpuffer, pH 6 mit 1 mmol/l EDTA. Anschließend wird der pH auf 7.8 eingestellt und mit 1mol/l Hydroxylamin-Lösung, pH 7,5 ad 2o mmol/l 1 Stunde bei 25 °C inkubiert. Zur Kopplung wird ein 5fach molarer Überschuß Benzoylecgoninmaleimidoethylamidhydrochlorid in Dimethylsulfoxid gelöst und unter Rühren zu der Lösung des mit Sulfhydrylgruppen modifizierten Kaninchen-IgG gegeben. Nach Inkubation bei 25 °C für 2 Stunden wird die Reaktion gestoppt durch die successive Zugabe von 0.1 mol/l Cystein-Lösung ad 1mmol/l unde 0.5 mol/l Jodacetamidlösung ad 5 mmol/l. Der Ansatz wird über Nacht gegen 0.1 mol/l Kaliumphosphatpuffer, pH 8.5 dialysiert und über Membranfiltration auf eine Proteinkonzentration von 10 mg/ml konzentriert. Danach wird das erhaltene Cocain-Polyhapten mit einem 8fach molarem Überschuß Biotinylcapronsäuresuccinimidylester, gelöst in Dimethylsulfoxid, biotinyliert.Der Ansatz wird gegen 20 mmol/l Natriumacetat, pH 4.3, dialysiert und über FPLC aufgereinigt.

### Beispiel 2

### a. Herstellung einer ersten Trägermatrix (Immunadsorber) mit einem Konjugat aus Anti-Cocain-Antikörper und alkalischer Phosphatase

Es wurde ein Vlies hergestellt, das aus 80 Teilen Polyesterfasern einer Faserfeinheit von 3,3 dtex und einer Faserlänge von 4 mm besteht, 20 Teilen Zellwolle mit einer Faserfeinheit von 1,7 dtex und einer Schnittlänge von 3 mm sowie 20 Teilen Polyvinylakoholfasern einer Schnittlänge von 4 mm. Die Faserstoffe Polyester, Zellwolle und Polyvinylalkohol wurden mit VE-Wasser bei einer Stoffdichte von 0,3% in Mischbütten aufgeschlagen bzw. vereinzelt. Der Faserstoffwurde anschließend auf ein umlaufendes Sieb gepumpt. Während das Fasergemisch entwässert bzw. das Wasser durch Vakuum abgesaugt wird, orientieren sich die Fasern auf der Siebseite und werden als Vlies mit einem Trockengehalt von ca. 20% über Trockenzylinder kontaktgetrocknet. Man erhält ein Vlies mit einem Flächengewicht von 80 g / m² und einer Dicke von 0.32 mm.

Aus diesem Vlies werden runde Scheiben mit einem Durchmesser von 18 mm gestanzt und mit je 20 µl einer Lösung der Konzentration 500 ng/ml des nach Beispiel 1 d. hergestellten Konjugates aus Anti-Cocain-Antikörper und alkalischer Phosphatase (freier erster Bindepartner) getränkt. Man erhält eine Ausführungsform des erfindungsgemäßen Immunadsorbers.

### b. Messung von Cocain aus der Gasphase

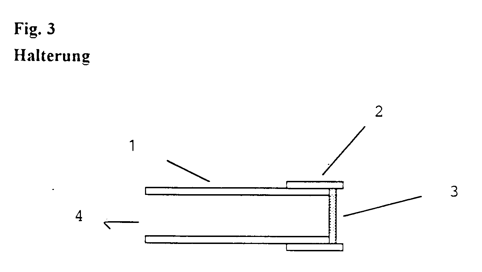

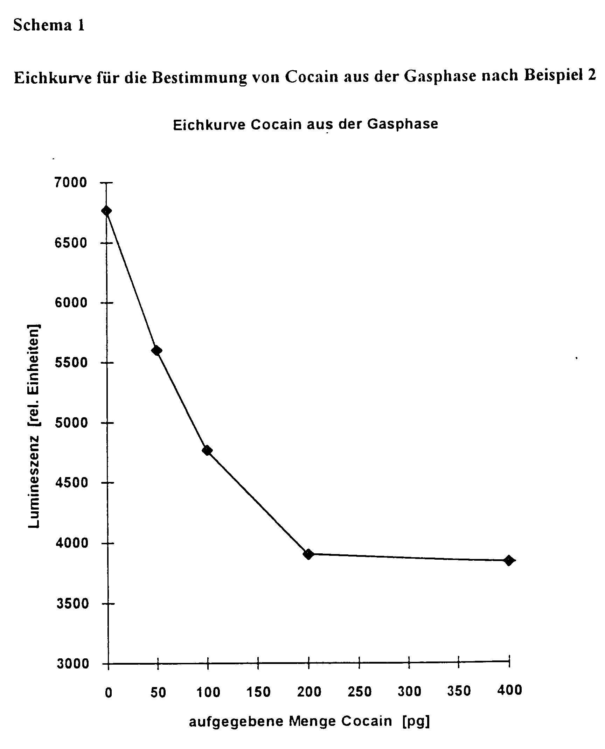

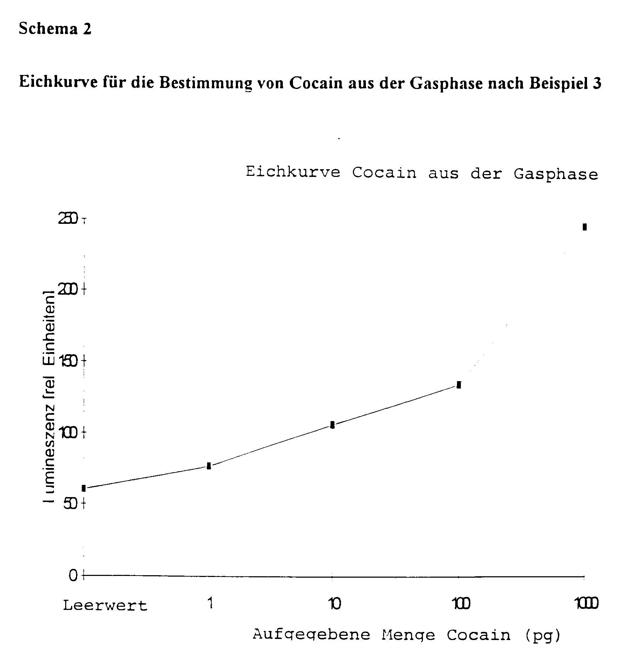

Die erfindungsgemäßen Immunadsorber werden in eine Halterung gem. Fig. 3 eingesetzt. Die Halterung mit dem eingesetzten Immunadsorber wird mit einer durchflußgeregelten Vakuumpumpe (Typ GSA 50-01) verbunden. Dabei bedeuten:
1) Kunststoffrohr
2) Führung für vliesförmiges Adsorbermaterial
3) Vliesförmiges Adsorbermaterial
4) Absaugrichtung zur Vakuumpumpe

Die Bereitstellung der Cocaingasphase erfolgt mittels eines Prüfstandes, in dem Stickstoffgas bei 20° C durch einen ebenfalls temperierten Bodenkörper von festem Cocain (freie Base) hindurchgeleitet wird. Die gesamte Gasführung des Prüfgasstandes erfolgt unter isothermen Bedingungen, um eine gleichbleibende Gasqualität zu gewährleisten. Das Vorliegen einer gleichbleibenden Gasqualität am Ausgang des Prüfstandes wird mittels apparativer Referenzanalytik (Einleiten des Prüfgases in mehrere hintereinandergeschaltete Lösungsmittelfallen, Analytik mit GC/MS) ermittelt. Bei der gegebenen Temperatur von 20°C wird eine Cocainkonzentration von ca. 2 ng/l erhalten, was der theoretisch zu erwartenden Sättigungskonzentration von Cocain für diese Temperatur entspricht (Lawrence et al., Can. J. Chem. 62, 1984). In einer zusätzlichen Verdünnungsstrecke wird das Cocaingas mit Stickstoff soweit verdünnnt, daß man 1% der Sättigungskonzentration, d.h. 20 pg/l oder 6,6 10⁻¹⁴ mol/l Cocain erhält.

An der Halterung wird ein Durchfluß von 5 l/min. eingestellt, wobei dieser mittels eines in die Gasführung eingesetzten Durchflußmessers (Rotameter) zusätzlich kontrolliert wird. Hierbei stellt sich ein Druckabfall von ca. 140 mbar ein. Mit der eingestellten Flußrate wird das Adsorbermaterial 30 sec., 1 min, 2 min, 4 min und 8 min lang am Ausgang des Gasprüfstandes mit dem verdünnten Cocaingas beaufschlagt. Dies entspricht Gesamtmengen von 50, 100, 200, 400 und 1000 ng Cocain, mit dem der Immunadsorber in Kontakt kommt.

Die Immunadsorber werden dann jeweils mit 400 µl Elutionspuffer (50 mmol/l HEPES, 0,9% NaCl, 0,05% Tween 20, pH 6,8) für 1 min geschüttelt.

Wells einer Mikrotiterplatte werden mit jeweils 100 µl einer Lösung von 10 ng Benzoylecgonin-[N'-biotinylaminocaproyl-(3,6-dioxa-8-aminooctyl)amid] in Elutionspuffer 60 min inkubiert und danach drei Mal mit Waschpuffer (0,9% NaCl + 0,05% Tween 20) gewaschen.

Jeweils 100 µl des Immunadsorbereluates, das freien ersten Bindepartner (Konjugat aus Anti-Cocain-Antikörper und alkalischer Phosphatase) und je nach der Menge des adsorbierten Cocains Komplex aus freiem ersten Bindepartner und Cocain enthält, werden in ein Well der Mikrotiterplatte gegeben, 60 min inkubiert und danach drei Mal mit Waschpuffer gewaschen.

Zur Detektion werden 100 µl Substratlösung (0,4 mmol/l CSBD di-Natrium 3-(Methoxyspiro{1,2-dioxetan-(5 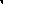 -chlor)-3,2 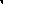 -tricyclo[3.3.1.1.^{3,7}]decan}phenylphosphat, 10% Sapphire-Enhancer in 0,1 M Diethanolamin, 1 mmol/l MgCl₂, pH 10,0) zugegeben und 60 sec. die Lumineszenz gemessen.

### Meßwerte:

| beaufschlagte Menge Cocain [pg] | Lumineszenz [rel. Einheiten / 60 sec.] |
|---|---|
| 0 | 6767,2 |
| 50 | 5602,3 |
| 100 | 4766,8 |
| 200 | 3901,7 |
| 400 | 3834,3 |
| 800 | 3871,0 |

Die erhaltene Eichkurve ist in Schema 1 gezeigt.

In einem Vergleichsversuch mit Einsatz einer genau eingewogenen Menge Cocain in den Immunoassay wurde bestimmt, daß die Ausbeute an gemessenem Cocain nach Adsorption und Elution bezogen auf beaufschlagtes Cocain 79% ist.

### Beispiel 3

### a. Trägeraufbau (Figur 1)

### Trägervlies (1)

Polyerstervlies der Firma Binzer, Hatzfeld, Bundesrepublik Deutschland. Es handelt sich um ein reines Polyestervlies, das mit 10% Kuralon verfestigt ist. Die Dicke beträgt 1,0 - 1,2 mm, die Saugkapazität 1800 ml/m².

### Erste Trägermatrix (Immunadsorber) (2)

Ein Mischvlies aus 80 Teilen Polyester und 20 Teilen Zellwolle, verfestigt mit 20 Teilen Kuralon, in einer Dicke von 0,32 mm und mit einer Saugkapazität von 500 ml/m² wird mit folgender Lösung getränkt und anschließend getrocknet: 3,8 10⁻⁸ mol/l nach Beispiel 1 d. hergestelltes Konjugat aus Anti-Cocain-Antikörper und alkalischer Phosphatase (freier erster Bindepartner), 3 mmol/l NaCl 1 mmol/l MgCl₂, 0,1 mmol/l ZnCl₂, 30 mmol/l Triethylamin pH 7.6.

### Zweite Trägermatrix (Abfangmatrix) (4)

Ein Vlies aus 100% Linters, verfestigt mit 2% Etadurin mit einer Dicke von 0.35 mm und einer Saugkapazität von 372 ml/m² wird mit folgender Lösung getränkt und anschließend getrocknet: 10 mmol/l Natriumphosphat pH 7.5, polymerisiertes Streptavidin 200 mg/l (Herstellung nach Beispiel 1c, EPS 0 331 127).
Das vorgetränkte Vlies wird anschließend nochmals getränkt und anschließend getrocknet: 10 mmol/l Natriumphosphat pH 7.5, 200 mg/l biotinyliertes Cocain-Polyhapten aus Beispiel 1 c.

### Dritte Trägermatrix (Nachweisfeld) (5)

Es wird ein Vlies aus 100% Linters, verfestigt mit 2% Etadurin mit einer Dicke von 0.35 mm und einer Saugkapazität von 372 ml/m² eingesetzt.

Die Trägermatrices werden gemaß Fig. 1 auf eine Trägerfolie von 6 mm Breite geklebt. Die Trägerfolie ist dabei mit einer Durchsaugöffnung (3) von 3 mm Durchmesser versehen, die mittig unter der ersten Trägermatrix (Immunadsorber) liegt.

### b. Messung von Cocain aus der Gasphase

Die Träger werden in eine Halterung eingesetzt, die eine Führung für ein Röhrchen mit O-Ring zur Abdichtung enthält. Das Röhrchen wird mit einer Vakuumpumpe wie in Beispiel 2 b beschrieben verbunden. Während der Sammlung der gasförmigen Probe wird das Röhrchen mit O-Ring unterhalb der ersten Trägermatrix so an die Trägerfolie gedruckt, daß das Probengas durch die erste Trägermatrix und das Loch in der Trägerfolie strömt.

Cocaingas einer Sättigung von 0.1% d.h. 2 pg/l oder 6.6 10⁻¹⁵ mol/l Cocain (hergestellt nach Beispiel 2 b) wird mit einer Flußrate von 2 l/min für 15 sec. (Gesamtmenge: 1 pg) und 2.5 min (Gesamtmenge: 10 pg) durch die erste Trägermatrix (Immunadsorber) des Trägers gesaugt.

Cocaingas einer Sättigung von 10% d.h. 200 pg/l oder 6.6 10⁻¹³ mol/l Cocain (hergestellt nach Beispiel 2 b) wird mit einer Flußrate von 1 l/min für 30 sec. (Gesamtmenge: 100 pg) und mit einer Flußrate von 10 l/min für 30 sec. (Gesamtmenge: 1000 pg) durch die erste Trägermatrix (Immunadsorber) des Trägers gesaugt.

Danach wird der Träger aus der Halterung entnommen und das Trägervlies 10 sec. in Pufferlösung (150 mmol/l NaCl, 50 mmol/l Kaliumphosphatpuffer pH 7.2) getaucht. Die von dem Trägervlies aufgenommene Flüssigkeit chromatographiert bis in die dritte Trägermatrix (Nachweisfeld). Aus der dritten Trägermatrix wird mit einem DIN 821 Locher ein rundes Teil ausgestanzt, mit der Vliesoberseite in ein Mikrotiterplatten-Well gelegt. In einem Lumineszenz-Mikrotiterplatten-Reader (Luminoscan) wird 100 µl Substratlösung (Lumiphos 530, Zubereitung von di-Natrium 3-(Methoxyspiro(1,2-dioxetan-3,2 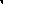 -tricyclo[3.3.1.1.^{3,7}]decan}phenylphosphat, Boehringer Mannheim) zugegeben. Die Lumineszenz wird über den Zeitraum von 10 min gemessen.

Zur Bestimmung des Leerwertes werden Träger in Pufferlösung getaucht, chromatographieren gelassen und analysiert, ohne daß vorher Cocain-Gas durchgesaugt wurde.

### Meßwerte:

| beaufschlagte Menge Cocain [pg] | Lumineszenz [rel. Einheiten / 60 sec.] |
|---|---|
| Leerwert | 60,46 |
| 1 | 76,86 |
| 10 | 106 |
| 100 | 134,4 |
| 1000 | 244,7 |

Die erhaltene Eichkurve ist in Schema 2 gezeigt.

### Beispiel 4

Anstelle des Cocaingases aus dem in Beispiel 2 b beschriebenen Prüfgasstand wird auf die erste Matrix (Immunadsorber) aus Beispiel 2 a bzw. den Träger aus Beispiel 3 Gas gesaugt, das Cocain-Partikel enthält. Die Probennanhme erfolgt in 1 cm Abstand über einer Polyethylenoberfläche, die zuvor mit einem pulverförmigen Gemisch aus 1 Teil Cocain und 1000 Teilen Lactose kontaminiert wurde, wobei 5 mg dieses Gemisches auf einem Oberflächenareal von 220 cm² verteilt wurden. Während der Probennahme wird die Oberfläche zusätzlich mit einem Preßluftstrom von 5 l/min aus einer Düse mit ca. 1 mm Durchmesser aus einem Abstand von ca. 1 cm angeblasen, um die Ablösung der partikulären Kontamination zu unterstützen. Bei allen Versuchen werden Meßwerte erhalten, die einer Cocainmenge >> 1 ng entsprechen.

## Patentansprüche

1. Verfahren zur Gewinnung eines in einer Gasphase enthaltenen Analyten durch immunologische Bindung des Analyten an einen in einer gas- und flüssigkeitspermeablen ersten Trägermatrix enthaltenen Bindepartner des Analyten, dadurch gekennzeichnet, daß
a) die analythaltige Gasphase mit der ersten Trägermatrix (Immunadsorber) in Kontakt gebracht wird,
b) der Analyt an den in der ersten Matrix enthaltenen und nicht an die Matrix gebundenen ersten Bindepartner gebunden wird und
c) der Komplex aus Analyt und erster Bindepartner sowie freier erster Bindepartner aus der ersten Matrix eluiert werden und
d) gegebenenfalls der Analyt aus dem Komplex freigesetzt wird.

2. Verfahren zum immunologischen Nachweis eines in einer Gasphase enthaltenen Analyten durch immunologische Bindung des Analyten an einen in einer gas- und flüssigkeitspermeablen ersten Trägermatrix enthaltenen Bindepartner des Analyten, dadurch gekennzeichnet, daß
a) die analythaltige Gasphase mit der ersten Trägermatrix in Kontakt gebracht wird,
b) der Analyt an den in der ersten Matrix enthaltenen und nicht an die Matrix gebundenen ersten Bindepartner gebunden wird,
c) der Komplex aus Analyt und erstem Bindepartner sowie freier erster Bindepartner aus der ersten Matrix eluiert werden und
d) der eluierte Komplex oder freie erste Bindepartner als Maß für die Anwesenheit oder Menge des vorhandenen Analyten bestimmt wird.

3. Verfahren nach Anspruch 2, dadurch gekennzeichnet, daß der erste Bindepartner mit einer Markierung versehen ist und die eluierte im Komplex gebundene oder ungebundene Markierung als Maß für die Menge des vorhandenen Analyten bestimmt wird.

4. Verfahren nach Anspruch 2, dadurch gekennzeichnet, daß der erste Bindepartner unmarkiert ist,
a) die eluierte Menge an erstem Bindepartner an einen markierten zweiten Bindepartner für den ersten Bindepartner gebunden wird
b) und die so gebundene Markierung als Maß für die Menge des vorhandenen Analyten bestimmt wird.

5. Verfahren nach Anspruch 4, dadurch gekennzeichnet, daß die Bindung des ersten Bindepartner an den zweiten Bindepartner in einer zweiten Trägermatrix und die Bestimmung der Markierung in einer dritten Trägermatrix erfolgt.

6. Verfahren nach den Ansprüchen 1 bis 5, dadurch gekennzeichnet, daß der Immunadsorber einen Flüssigkeitsgehalt von 10 bis 90 % besitzt.

7. Verfahren nach Anspruch 6, dadurch gekennzeichnet, daß der Analyt in einem ersten Schritt an eine im wesentlichen trockene Matrix unspezifisch adsorbiert wird, in einem zweiten Schritt Flüssigkeit zugegeben wird, wodurch die Immunreaktion zwischen Analyt und markiertem Bindepartner erfolgt.

8. Verfahren nach Anspruch 6 oder 7, dadurch gekennzeichnet, daß die Flüssigkeit Wasser mit einem Gehalt von bis zu 30 Gew.-% organischen substanzen als Lösevermittler und/oder 0,01 bis 1 % Detergens ist.

9. Vorrichtung zur Gewinnung und/oder zum immunologischen Nachweis eines in einer Gasphase enthaltenen Analyten, enthaltend
a) eine gas- und flüssigkeitspermeable Trägermatrix. in der ein erster Bindepartner des Analyten in eluierbarer Form enthalten ist.
b) ein Auffangsystem für ein solches Eluat, in welchem der Komplex aus Analyt und erstem Bindepartner oder ungebundener erster Bindepartner gegebenenfalls in einer Folgeraktion bestimmt oder isoliert werden kann.

10. Vorrichtung nach Anspruch 9, dadurch gekennzeichnet. daß die Trägermatrix eine Gasdurchlässigkeit von 10ml/min bis 10l/min besitzt.

## Claims

1. Method of obtaining an analyte contained in a gas phase by immunologically binding the analyte to a binding partner thereof contained in a gas and liquid-permeable first carrier matrix, characterized in that
a) the analyte-containing gas phase is brought into contact with the first carrier matrix (immune adsorber),
b) the analyte is bound to the first binding partner which is contained in the first matrix and not bound to the matrix, and
c) the complex consisting of analyte and free first binding partner is eluted from the first matrix, and
d) the analyte can be released from the complex.

2. Method of immunologically detecting an analyte contained in a gas phase by immunologically binding the analyte to a binding partner thereof contained in a gas- and liquid-permeable first carrier matrix, characterized in that
a) the analyte-containing gas phase is brought into contact with the first carrier matrix (immune adsorber),
b) the analyte is bound to the first binding partner which is contained in the first matrix and not bound to the matrix, and
c) the mixture consisting of complex of analyte and first binding partner and free first binding partner is eluted from the first matrix
d) the eluted complex or free first binding partner is determined as a measure for the amount of analyte present.

3. Method according to claim 2, characterized in that the first binding partner is provided with a label, and the eluted label which is bound in the complex or not bound is determined as a measure for the amount of analyte which is present.

4. Method according to claim 2, characterized in that the first binding partner is unlabeled,
a) the eluted amount of first binding partner is bound to a labeled second binding partner for the first binding partner
b) and the so bound label is determined as a measure for the amount of analyte present.

5. Method according to claim 4, characterized in that the binding of the first binding partner to the second binding partner is accomplished in a second carrier matrix and the determination of the label in a third carrier matrix.

6. Method according to claims 1 to 5, characterized in that the immune adsorber has a liquid content of 10 to 90 %.

7. Method according to claim 6, characterized in that, in a first step, the analyte is nonspecifically adsorbed to an essentially dry matrix; in a second step, liquid is added allowing the immune reaction between analyte and labeled binding partner to occur.

8. Method according to claims 6 or 7, characterized in that the liquid is water with a content of up to 30 wt.-% of organic substances as dissolving agents and/or 0.01 to 1 % detergent.

9. Device for obtaining and/or immunologically detecting an analyte contained in a gas phase, comprising
a) a gas- and liquid-permeable carrier matrix in which a first binding partner of the analyte is contained in elutable form,
b) a capture system for such an eluate in which the complex consisting of analyte and first binding partner or nonbound first binding partner can be determined or isolated in a subsequent reaction.

10. Device according to claim 9, characterized in that the carrier matrix has a gas permeability of 10 ml/min up to 10 l/min.

## Revendications

1. Procédé pour l'obtention d'un analyte contenu dans une phase gazeuse par liaison immunologique de l'analyte à un premier partenaire de liaison de l'analyte contenu dans une première matrice de support perméable aux gaz et aux liquides, caractérisé en ce que
a) on amène la phase gazeuse contenant l'analyte en contact avec la première matrice de support (agent immuno-adsorbant),
b) on lie l'analyte au premier partenaire de liaison contenu dans la première matrice et non lié à la matrice, et
c) on élue de la première matrice le complexe de l'analyte et du premier partenaire de liaison, ainsi que le premier partenaire de liaison libre, et
d) on libère le cas échéant l'analyte du complexe.

2. Procédé pour l'examen immunologique d'un analyte contenu dans une phase gazeuse par liaison immunologique de l'analyte à un partenaire de liaison de l'analyte contenu dans une première matrice de support perméable aux gaz et aux liquides, caractérisé en ce que
a) on amène la phase gazeuse contenant l'analyte en contact avec la première matrice de support,
b) on lie l'analyte au premier partenaire de liaison contenu dans la première matrice et non lié à la matrice,
c) on élue de la première matrice le complexe de l'analyte et du premier partenaire de liaison, ainsi que le premier partenaire de liaison libre, et
d) on détermine le complexe ou le premier partenaire libre élué comme mesure pour la présence de l'analyte ou pour la mesure de sa quantité.

3. Procédé selon la revendication 2, caractérisé en ce qu'on munit d'un marquage le premier partenaire de liaison et on détermine le marquage élué lié dans le complexe ou non lié comme mesure pour la quantité de l'analyte présent.

4. Procédé selon la revendication 2, caractérisé en ce qu'on ne munit pas d'un marquage le premier partenaire de liaison,
a) on lie la quantité éluée du premier partenaire de liaison à un deuxième partenaire de liaison marqué pour le premier partenaire de liaison,
b) et on détermine le marquage lié de cette manière comme mesure pour la quantité de l'analyte présent.

5. Procédé selon la revendication 4, caractérisé en ce que la liaison du premier partenaire de liaison au deuxième partenaire de liaison a lieu dans une deuxième matrice de support et la détermination du marquage a lieu dans une troisième matrice de support.

6. Procédé selon les revendications 1 à 5, caractérisé en ce que la teneur en liquide de l'agent d'immuno-adsorption s'élève de 10 à 90% en poids.

7. Procédé selon la revendication 6, caractérisé en ce qu'on soumet l'analyte à une adsorption non spécifique dans une première étape sur une matrice essentiellement sèche, on ajoute du liquide dans une deuxième étape donnant lieu à une immunoréaction entre l'analyte et le partenaire de liaison marqué.

8. Procédé selon la revendication 6 ou 7, caractérisé en ce que le liquide est de l'eau possédant une teneur, jusqu'à concurrence de 30% en poids, en substances organiques à titre d'agent de solubilisation et/ou en détergent à concurrence de 0,01 à 1%.

9. Dispositif pour l'obtention et/ou pour l'examen immunologique d'un analyte contenu dans une phase gazeuse, contenant
a) une matrice de support perméable aux gaz et aux liquides dans laquelle est contenu un premier partenaire de liaison de l'analyte sous une forme éluable,
b) un système de récupération pour un tel éluat dans lequel le complexe de l'analyte et du premier partenaire de liaison ou le premier partenaire de liaison non lié peut être le cas échéant déterminé ou isolé dans une réaction ultérieure.

10. Dispositif selon la revendication 9, caractérisé en ce que la matrice de support possède une perméabilité aux gaz de 10 ml/min à 10 l/min.
